# EUROPEAN PATENT SPECIFICATION

(11) **EP 1 974 007 B1**
(45) Date of publication and mention of the grant of the patent: **11.11.2009**
(21) Application number: 06831484.8
(22) Date of filing: 27.12.2006
(51) Int. Cl.: C12M 1/06

(54) **FERMENTATION VESSELS**
FERMENTATIONSGEFÄSSE
CUVES DE FERMENTATION

(30) Priority: 14.01.2006 GB 0600729
(43) Date of publication of application: 01.10.2008
(73) Proprietor: John Crane UK Limited, Slough SL1 4LU (GB)
(72) Inventor: MAYER, Hans-Peter, D-36041 Fulda (DE)
(74) Representative: Watts, Peter Graham
(86) International application number: PCT/GB2006/004933
(87) International publication number: WO 2007/080369

(56) References cited:
- EP-A2- 1 479 758
- DE-A1- 19 505 257
- US-A- 5 954 341

## Description

The present invention relates to fermentation vessels and in particular an apparatus for the aeration of submerged microbial or cell cultures, for biological purposes.

In such vessels a sufficient supply of oxygen is essential for the growth of aerobic micro-organisms as well as cell cultures and is therefore critical to obtain economic product yields in biotechnology using submerse fermentation processes. Standard oxygen supply of submerse fermentation processes is performed by introducing an oxygen containing gas via a stationary aeration ring at the bottom of a fermentation vessel. The efficiency of the fermentation process depends on the rate of transfer of oxygen across the gas/liquid interface to the liquid growth medium. This transfer rate depends mainly on the total interface surface, which depends on the size of the bubbles of oxygen introduced into the vessel, the smaller the bubbles, the higher the surface interface.

With conventional fermentation apparatus, the gas bubbles introduced into the bottom of the vessel, have a tendency to coagulate into larger bubbles, as the bubbles travel up the vessel. Anticoagulation chemicals may be used to reduce this effect. However such chemicals may adversely effect the product quality.

Hitherto an alternative solution to the problem of coagulation has been the use of mechanical stirrers to brake up larger bubbles. To be effective such stirrers require high speed mixing which may be detrimental to cell cultures used in modern biotechnology. Furthermore rapid stirring of the biological mass will induce heating, requiring cooling apparatus in order to maintain appropriate operating temperatures and therefore results in high energy costs.

European Patent Specification EP1479758 discloses a fermentation apparatus in which oxygen is introduced into the fermentation vessel via a hollow agitator shaft, at a plurality of distinct levels within the vessel, low speed stirring means being provided at each level. This construction enables an increase in the total gas/liquid interface to be achieved, at low stirring speeds. However this solution is relatively expensive and is only suitable for medium to large scale fermentation vessels.

The present invention provides a cost effective solution to the problem of coagulation, which is suitable for small scale fermentation vessels.

Hereinafter the term processing gas shall mean a pure gas or a gas mixture that contains at least one component, said pure gas or at least one component being capable of being metabolised by micro-organisms in a fermentation process.

According to one aspect of the present invention, a fermentation apparatus comprises, a fermentation vessel having a low speed stirrer, the stirrer comprising a shaft entering the vessel through an aperture in the bottom of the vessel, one or more stirring elements being mounted on the shaft for rotation therewith, the or each stirring element being disposed within the vessel; and sealing means being provided to seal the gap between the shaft and aperture, said sealing means having a cylindrical housing attached to the bottom of the vessel, externally thereof and surrounding the shaft as it passes through the bottom of the vessel; characterised in that, first and second seal assemblies acting between the shaft and housing, are provided at axially spaced locations, to define a chamber therebetween, an inlet opening into said chamber for connection of the chamber to a supply of processing gas, the first seal assembly being located between the fermentation vessel and the chamber comprising a first sealing ring mounted on the shaft for rotation therewith and a second sealing ring mounted in fixed rotational relationship to the housing, the first and second sealing rings being movable axially relative to one another and being resiliently biased towards one another, so that a sealing face of the first sealing ring may be biased into engagement with a sealing face of the second sealing ring, the sealing face of one of the first and second sealing rings having grooves, which will provide hydrodynamic separation of the sealing faces upon rotation of the shaft, allowing processing gas in the chamber to flow inwardly across the sealing faces and into the fermentation vessel, the grooves being bounded by a continuous dam formation adjacent the outer periphery of the sealing ring, so that when the sealing faces engage, the dam formation on the one sealing face will sealingly engage the other sealing face.

The faces of the first and second sealing rings are configured to provide a hydrostatic and/or hydrodynamic separation between the sealing faces sufficient to allow an optimum passage of processing gas into the fermentation vessel to fulfil the requirements of the micro-organisms in use.

With the apparatus described above, when the shaft is rotating, hydrostatic/hydrodynamic effects will cause the seal faces to move apart, gas flowing between the seal faces from the chamber into the fermentation vessel. The processing gas which is fed to the chamber defined by the seal housing is thus fed into the fermentation vessel. Due to the narrow gap that is formed between the sealing faces and high shear forces, very small bubbles are formed in this manner and the centrifugal forces generated by rotation of the seat efficiently mix the bubbles with the liquid in the fermentation vessel. Both of these effects increase the gas/liquid interface in the fermentation vessel and therefore the transfer rate of the processing gas, even at low speeds. The seal faces may also be moved apart by hydrostatic forces, when pressure in the chamber is above a predetermined value, so that the processing gas may be fed to the fermentation vessel while there is sufficient pressure in the chamber, even though the shaft is not rotating. When the shaft stops rotating and the pressure of fluid in the chamber falls below the predetermined value, the resilient biasing of the seal rings will force the dam formation on one ring to sealingly engage the other ring to form a fluid tight seal.

The use of a hydrodynamic seal in accordance with the present invention also has the advantage that when the shaft is rotating the sealing faces are separated so that there will be no wear of the sealing faces, the debris of which may otherwise contaminate the product of the fermentation process.

According to a preferred embodiment of the invention, deflecting formations are provided on the external circumference of the seat adjacent the sealing face thereof, in order to enhance distribution of the bubbles in the fermentation vessel.

The invention is now described, by way of example only, with reference to the accompanying drawings, in which:-
Figure 1 is a diagrammatic illustration in sectional elevation, of a fermentation apparatus in accordance with the present invention;
Figure 2 shows a modification to the embodiment illustrated in figure 1; and
Figures 3 and 4 show further modifications to the apparatus shown in figure 1.

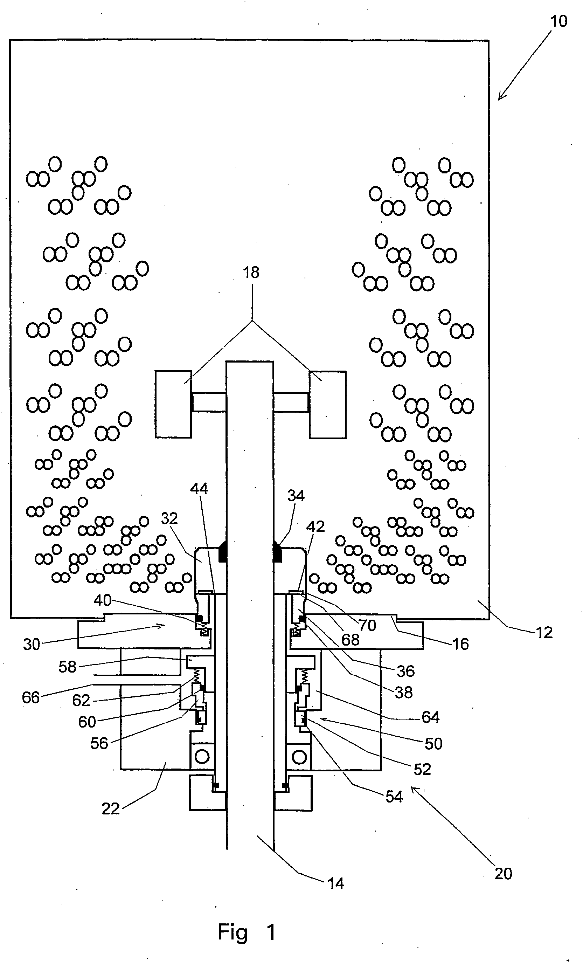

As shown in figure 1 a fermentation apparatus 10 comprises a fermentation vessel 12 in which, for example, a liquid microbial growth culture is contained. A shaft 14 is rotatably mounted through an aperture 16 in the bottom of the vessel 12. Mixing elements 18 are mounted on the shaft 14, within the vessel 12 and the shaft 14 is connected to drive means (not shown) externally of the vessel 12, by which the shaft 14 may be rotated at low speeds, to provide low energy stirring of the microbial growth culture.

The aperture 16 is closed by a hydrodynamic seal means 20, to provide a seal between the shaft 14 and vessel 12. The seal means 20 comprises a cylindrical housing 22 secured to the bottom of the vessel 12 and surrounding the shaft 14 as it enters the vessel 12. A first seal assembly 30 has a first sealing ring 32 which is mounted for rotation on shaft 14, within the fermentation vessel 12. The first sealing ring 32 is sealed with respect to the shaft 14 by elastomeric ring 34. A second sealing ring 36 is mounted on the housing 22 adjacent the inner end thereof. The second sealing ring 36 is secured to the housing to prevent rotation thereof, but is moveable axially of the housing 22. A sealing ring 38 slidably seals the second sealing ring 36 to the housing 22 and a series of angularly spaced helical compression springs 40 act axially between the housing 22 and second sealing ring 36, to urge a sealing face 42 of the second sealing ring 36, into sealing engagement with a sealing face 44 of the first sealing ring 32.

A second seal assembly 50 is mounted adjacent the outer end of housing 22. The second seal assembly 50 has a seat 52 mounted in fixed rotational and axial relationship to the housing 22 and sealed thereto by means of an elastomeric ring 54. A mating ring 56 is mounted on a carrier ring 58 on the shaft 14, the mating ring being rotationally fixed to the shaft but movably axially thereof, the mating ring being slidably sealed to the carrier ring 58 by sealing ring 60. A series of angularly spaced helical compression springs 62 act axially between the carrier ring 58 and mating ring 56 urging it into sealing engagement with the seat 52.

The first and second seal assemblies 30, 50 thereby define a chamber 64 externally of the fermentation vessel 12. An inlet 66 is provided to the chamber, the inlet 66 being connected to a source of processing gas.

The sealing face 44 of the first sealing ring 32 has a series of angularly spaced helical grooves 68. The grooves 68 extend outwardly, from a diameter radially inwardly of the internal diameter of the sealing face 42 of the second sealing ring 36 to a diameter radially inwardly of the external diameter of the sealing faces 42 and 44, the grooves 68 being inclined at an obtuse angle to the direction of rotation of the shaft 14. In this manner a continuous dam formation 70 is provided on the sealing face 44, which when the shaft 14 is stationary and pressure in the chamber 64 is below a predetermined value, is engaged by sealing face 42, to prevent leakage of the microbial growth culture, from the fermentation vessel 12.

When the shaft 14 rotates, processing gas in the chamber 64 is forced into the grooves 68, creating a high pressure zone adjacent the dam formation 70 and forcing the sealing faces 42, 44 apart to form a narrow gap between the sealing faces 42,44. The narrow gap permits the gas to flow between the sealing faces creating small bubbles in the microbial growth culture, the bubbles being distributed throughout the vessel 12 by the centrifugal action of the first sealing ring 32. Even when the shaft 14 is stationary, provided that the pressure of gas in chamber 64 is above a predetermined value, for example 2.5 bar, hydrostatic forces will open the seal faces 42,44 and allow gas to enter the vessel 12.

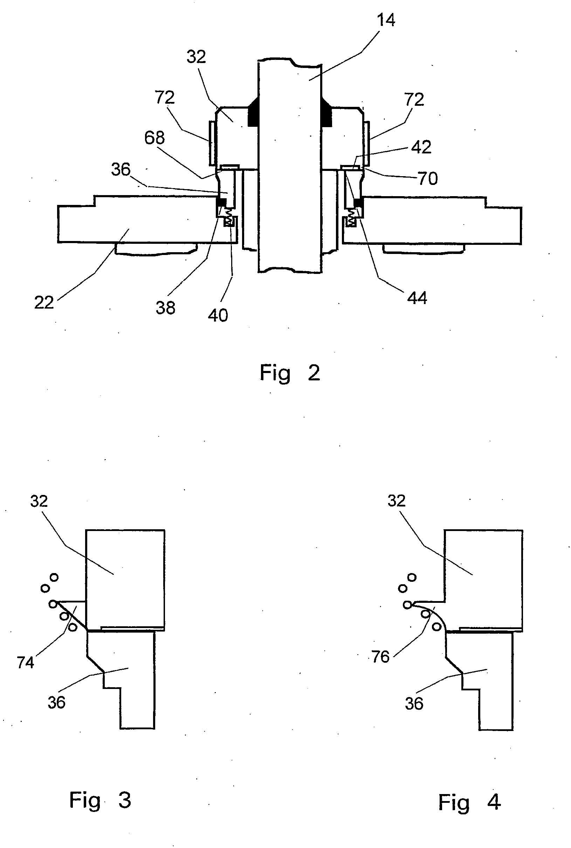

As illustrated on figure 2, axially extending vanes 72 may be provided of the circumferential surface of the first sealing ring 32 to augment or replace the mixing elements 18, to enhance the centrifugal effect and improve the distribution of the bubbles throughout the growth culture.

In the embodiments illustrated in figures 3 and 4 wedge shaped annular formations 74, 76 are provided on the circumferential surface of the first sealing ring 32, adjacent the sealing face 44. The wedge formations 74, 76 will increase the diameter at which the bubbles detach from the first sealing ring 32 and thus the distribution of the bubbles throughout the growth culture. The wedge 74, 76 formations may be formed integrally of the first sealing ring 32 or may be formed separately of, for example, a low friction material, such as PTFE.

In order to inhibit gas bubbles from clinging to the sealing faces, the first sealing ring32 and second sealing ring 36 are preferably made of low surface energy materials, or the sealing faces thereof may be coated with materials having low surface energy. Such materials include flurocarbons such as PTFE, or diamond and diamond like materials.

Various modifications may be included without departing from the invention. For example, while in the above embodiment, grooves 68 in the sealing face 44 provide hydrostatic as well as hydrodynamic separation, alternatively, the sealing faces 42,44 may be angled to provide hydrostatic separation of the faces 42,44, when pressure in the chamber 64 exceeds a predetermined value.

## Claims

1. A fermentation apparatus (10) comprising, a fermentation vessel (12) having a low speed stirrer, the stirrer comprising a shaft (14) mounted co-axially of the vessel (12) and entering the vessel (12) through an aperture (16) in the bottom of the vessel (12), one or more stirring elements (18;72) being mounted on the shaft (14) for rotation therewith, the or each stirring element (18;72) being disposed within the vessel (12); and sealing means (20) being provided to seal the gap between the shaft (14) and aperture (16), said sealing means has a cylindrical housing (22) attached to the bottom of the vessel (12), externally thereof and surrounding the shaft (14) as it passes through the bottom of the vessel (12); **characterised in that**, first and second seal assemblies (30,50) acting between the shaft (14) and housing (22), are provided at axially spaced locations, to define a chamber (64) therebetween, an inlet (66) opening into said chamber (64) for connection of the chamber (64) to a supply of processing gas, the first seal assembly (30) being located between the fermentation vessel (12) and the chamber (64) comprising a first sealing ring (32) mounted on the shaft (14) for rotation therewith and a second sealing ring (36) mounted in fixed rotational relationship the housing (22), the first and second sealing rings (32,36) being movable axially relative to one another and being resiliently biased towards one another, so that a sealing face (44) of the first sealing ring (32) may be biased into engagement with a sealing face (42) of the second sealing ring (36), the sealing face (42,44) of one of first and second sealing rings (32,36) having grooves (68), which will provide hydrodynamic separation of the sealing faces upon rotation of the shaft (14), allowing processing gas in the chamber (64) to flow inwardly across the sealing faces (42,44) and into the fermentation vessel (12), the grooves (68) being bounded by a continuous dam formation (70) adjacent the outer periphery of the sealing ring (32,36), so that when sealing face (42) engages sealing face (44) the dam formation (70) on the one sealing face (42,44) will sealingly engage the other sealing face (44,42).

2. A fermentation apparatus (10) in accordance with claim 1 **characterised in that** when the shaft is stationary, the sealing faces (42,44) are forced apart hydrostatically, when pressure in the chamber (64) is above a predetermined value.

3. A fermentation apparatus (10) in accordance with claim 1 or 2 **characterised in that** the grooves (68) provide hydrostatic separation.

4. A fermentation apparatus (10) in accordance with claim 2 **characterised in that** the seal faces (42,44) are angled to provide hydrostatic separation.

5. A fermentation apparatus in accordance with any one of the preceding claims **characterised in that** the sealing faces of the first and/or second sealing ring (32,36) are formed from material having a low surface energy.

6. A fermentation apparatus (10) in accordance with any one of claims 1 to 5 **characterised in that** the second sealingring (36) or the first sealing ring (32) of the first seal assembly (30) has a series of angularly spaced helical grooves (68), the grooves (68) being inclined at an obtuse angle to the direction of rotation of the shaft (14), the inner ends of the grooves (68) being exposed to processing gas within the chamber (64).

7. A fermentation apparatus (10) in accordance with any one of the preceding claims **characterised in that** formations (72,74,76) are provided on the first sealing ring (32) of the first seal assembly (30) to enhance distribution of processing gas bubbles through a liquid within the vessel (12).

8. A fermentation apparatus (10) in accordance with claim 7 **characterised in that** angularly spaced, axially extending vanes (72) are provided on the circumferential surface of the first sealing ring (32), to augment or replace mixing elements (18).

9. A fermentation apparatus (10) in accordance with claim 7 or 8 **characterised in that** a wedge shaped annular formation (74,76) is provided on the circumferential surface of the first sealing ring (32), the annular formation (74,76) being located adjacent the sealing face (44) of the seat (32).

10. A fermentation apparatus (10) in accordance with claim 9 **characterised in that** the annular formation (76) is formed integrally of the first sealing ring(32).

11. A fermentation apparatus (10) in accordance with claim 10 **characterised in that** the annular formation (74) is formed separately of the first sealing ring (32), from a low friction material.

## Patentansprüche

1. Fermentationsapparat (10), der ein Fermentationsgefäß (12) mit einem langsamlaufenden Rührwerk aufweist, wobei das Rührwerk aufweist: eine Welle (14), die koaxial zum Gefäß (12) montiert ist und in das Gefäß (12) durch eine Öffnung (16) im Boden des Gefäßes (12) eintritt; ein oder mehrere Rührelemente (18; 72), die auf der Welle (14) für eine Drehung damit montiert sind, wobei das oder jedes Rührelement (18; 72) innerhalb des Gefäßes (12) angeordnet ist; und eine Dichtungseinrichtung (20), die bereitgestellt wird, um den Spalt zwischen der Welle (14) und der Öffnung (16) abzudichten, wobei die Dichtungseinrichtung ein am Boden des Gefäßes (12) befestigtes zylindrisches Gehäuse (22) aufweist, extern davon und die Welle (14) umgebend, während sie durch den Boden des Gefäßes (12) hindurchgeht; **dadurch gekennzeichnet, dass** eine erste und eine zweite Dichtungsbaugruppe (30, 50), die zwischen der Welle (14) und dem Gehäuse (22) wirken, an axial beabstandeten Stellen bereitgestellt werden, um eine Kammer (64) dazwischen zu definieren, wobei sich ein Einlass (66) in die Kammer (64) für einen Anschluss der Kammer (64) an eine Zuführung von Verarbeitungsgas öffnet, wobei die erste Dichtungsbaugruppe (30), die zwischen dem Fermentationsgefäß (12) und der Kammer (64) angeordnet ist, einen ersten Dichtungsring (32), der auf der Welle (14) für eine Drehung damit montiert ist, und einen zweiten Dichtungsring (36) aufweist, der in einer konstanten Rotationsbeziehung zum Gehäuse (22) montiert ist, wobei der erste und der zweite Dichtungsring (32, 36) axial relativ zueinander beweglich und elastisch in Richtung zueinander vorgespannt sind, so dass eine Dichtungsfläche (44) des ersten Dichtungsringes (32) in Anlage mit einer Dichtungsfläche (42) des zweiten Dichtungsringes (36) vorgespannt werden kann, wobei die Dichtungsfläche (42, 44) eines von erstem und zweitem Dichtungsring (32, 36) Rillen (68) aufweist, die eine hydrodynamische Trennung der Dichtungsflächen bei der Drehung der Welle (14) bewirken werden, wodurch das Strömen von Verarbeitungsgas in die Kammer (64) nach innen über die Dichtungsflächen (42, 44) und in das Fermentationsgefäß (12) gestattet wird, wobei die Rillen (68) durch eine kontinuierliche Dammausbildung (70) benachbart dem äußeren Umfang des Dichtungsringes (32, 36) begrenzt werden, so dass, wenn die Dichtungsfläche (42) mit der Dichtungsfläche (44) in Anlage kommt, die Dammausbildung (70) auf der einen Dichtungsfläche (42, 44) abdichtend mit der anderen Dichtungsfläche (44, 42) in Anlage kommen wird.

2. Fermentationsapparat (10) nach Anspruch 1, **dadurch gekennzeichnet, dass**, wenn die Welle stationär ist, die Dichtungsflächen (42, 44) hydrostatisch auseinandergedrückt werden, wenn der Druck in der Kammer (64) über einem vorgegebenen Wert ist.

3. Fermentationsapparat (10) nach Anspruch 1 oder 2, **dadurch gekennzeichnet, dass** die Rillen (68) eine hydrostatische Trennung bewirken.

4. Fermentationsapparat (10) nach Anspruch 2, **dadurch gekennzeichnet, dass** die Dichtungsflächen (42, 44) winkelig sind, um eine hydrostatische Trennung zu bewirken.

5. Fermentationsapparat (10) nach einem der vorhergehenden Ansprüche, **dadurch gekennzeichnet, dass** die Dichtungsflächen des ersten und/oder des zweiten Dichtungsringes (32, 36) aus einem Material mit einer niedrigen Oberflächenenergie gebildet werden.

6. Fermentationsapparat (10) nach einem der Ansprüche 1 bis 5, **dadurch gekennzeichnet, dass** der zweite Dichtungsring (36) oder der erste Dichtungsring (32) der ersten Dichtungsbaugruppe (30) eine Reihe von winkelig beabstandeten spiralförmigen Rillen (68) aufweist, wobei die Rillen (68) unter einem stumpfen Winkel zur Richtung der Drehung der Welle (14) geneigt sind, wobei die inneren Enden der Rillen (68) dem Verarbeitungsgas innerhalb der Kammer (64) ausgesetzt sind.

7. Fermentationsapparat (10) nach einem der vorhergehenden Ansprüche, **dadurch gekennzeichnet, dass** Ausbildungen (72, 74, 76) am ersten Dichtungsring (32) der ersten Dichtungsbaugruppe (30) vorhanden sind, um die Verteilung der Blasen des Verarbeitungsgases durch eine Flüssigkeit innerhalb des Gefäßes (12) zu verbessern.

8. Fermentationsapparat (10) nach Anspruch 7, **dadurch gekennzeichnet, dass** winkelig beabstandete sich axial erstreckende Flügel (72) auf der Umfangsfläche des ersten Dichtungsringes (32) vorhanden sind, um die Mischelemente (18) zu vergrößern oder zu ersetzen.

9. Fermentationsapparat (10) nach Anspruch 7 oder 8, **dadurch gekennzeichnet, dass** eine keilartige, ringförmige Ausbildung (74, 76) auf der Umfangsfläche des ersten Dichtungsringes (32) vorhanden ist, wobei die ringförmige Ausbildung (74, 76) benachbart der Dichtungsfläche (44) des Sitzes (32) angeordnet ist.

10. Fermentationsapparat (10) nach Anspruch 9, **dadurch gekennzeichnet, dass** die ringförmige Ausbildung (76) zusammenhängend mit dem ersten Dichtungsring (32) gebildet wird.

11. Fermentationsapparat (10) nach Anspruch 10, **dadurch gekennzeichnet, dass** die ringförmige Ausbildung (74) separat vom ersten Dichtungsring (32) aus einem reibungsarmen Material gebildet wird.

## Revendications

1. Appareil de fermentation (10) comprenant une cuve de fermentation (12) possédant un agitateur à vitesse lente, l'agitateur comprenant un arbre (14) monté coaxialement à la cuve (12) et pénétrant dans la cuve (12) à travers une ouverture (16) dans le fond de la cuve (12), un ou plusieurs éléments d'agitation (18 ; 72) étant montés sur l'arbre (14) pour tourner avec celui-ci, le ou chaque élément d'agitation (18 ; 72) étant disposé à l'intérieur de la cuve (12) ; et des moyens d'étanchéité (20) étant prévus pour étanchéifier l'espace entre l'arbre (14) et l'ouverture (16), lesdits moyens d'étanchéité possédant un logement cylindrique (22) fixé sur le fond de la cuve (12), sur le côté extérieur de celle-ci, et entourant l'arbre (14) tandis qu'il traverse le fond de la cuve (12) ; **caractérisé en ce qu'**un premier ensemble d'étanchéité (30) et un second ensemble d'étanchéité (50), agissant entre l'arbre (14) et le logement (22), sont prévus en des emplacements axialement espacés, pour définir entre eux une chambre (64), une entrée (66) s'ouvrant dans ladite chambre (64) pour le raccordement de la chambre (64) à une alimentation en gaz de traitement, le premier ensemble d'étanchéité (30), situé entre la cuve de fermentation (12) et la chambre (64), comprenant une première bague d'étanchéité (32) montée sur l'arbre (14) pour tourner avec celui-ci et une deuxième bague d'étanchéité (36) montée solidarisée en rotation par rapport au logement (22), la première bague d'étanchéité (32) et la deuxième bague d'étanchéité (36) étant mobiles axialement l'une par rapport à l'autre et étant précontraintes élastiquement l'une vers l'autre, de sorte qu'une surface d'étanchéité (44) de la première bague d'étanchéité (32) peut être précontrainte en engagement avec une surface d'étanchéité (42) de la deuxième bague d'étanchéité (36), la surface d'étanchéité (42, 44) de la première bague d'étanchéité (32) ou de la deuxième bague d'étanchéité (36) possédant des rainures (68) qui assurent une séparation hydrodynamique des surfaces d'étanchéité lors de la rotation de l'arbre (14), permettant au gaz de traitement présent dans la chambre (64) de s'écouler vers l'intérieur entre les surfaces d'étanchéité (42, 44) et dans la cuve de fermentation (12), les rainures (68) étant délimitées par une formation (70) formant une barrière continue au voisinage de la périphérie extérieure de la bague d'étanchéité (32, 36), de sorte que, lorsque la surface d'étanchéité (42) engage la surface d'étanchéité (44), la formation formant barrière (70) présente sur l'une des surfaces d'étanchéité (42, 44) engage en étanchéité l'autre surface d'étanchéité (44, 42).

2. Appareil de fermentation (10) selon la revendication 1, **caractérisé en ce que**, lorsque l'arbre est immobile, les surfaces d'étanchéité (42, 44) sont forcées hydrostatiquement en éloignement l'une de l'autre lorsque la pression dans la chambre (64) est supérieure à une valeur prédéterminée.

3. Appareil de fermentation (10) selon la revendication 1 ou 2, **caractérisé en ce que** les rainures (68) assurent la séparation hydrostatique.

4. Appareil de fermentation (10) selon la revendication 2, **caractérisé en ce que** les surfaces d'étanchéité (42, 44) sont angulaires, afin d'assurer la séparation hydrostatique.

5. Appareil de fermentation selon l'une quelconque des revendications précédentes, **caractérisé en ce que** les surfaces d'étanchéité de la première et/ou de la deuxième bagues d'étanchéité (32, 36) sont constituées d'un matériau ayant une faible énergie superficielle.

6. Appareil de fermentation (10) selon l'une quelconque des revendications 1 à 5, **caractérisé en ce que** la deuxième bague d'étanchéité (36) ou la première bague d'étanchéité (32) du premier ensemble d'étanchéité (30) possède une série de rainures hélicoïdales (68) angulairement espacées, les rainures (68) étant inclinées sous un angle obtus par rapport à la direction de rotation de l'arbre (14), les extrémités intérieures des rainures (68) étant exposées au gaz de traitement présent dans la chambre (64).

7. Appareil de fermentation (10) selon l'une quelconque des revendications précédentes, **caractérisé en ce que** des formations (72, 74, 76) sont prévues sur la première bague d'étanchéité (32) du premier ensemble d'étanchéité (30) pour améliorer la répartition des bulles de gaz de traitement dans un liquide présent dans la cuve (12).

8. Appareil de fermentation (10) selon la revendication 7, **caractérisé en ce que** des ailettes (72) angulairement espacées, s'étendant axialement, sont prévues sur la surface circonférentielle de la première bague d'étanchéité (32) pour agrandir ou remplacer les éléments mélangeurs (18).

9. Appareil de fermentation (10) selon la revendication 7 ou 8, **caractérisé en ce qu'**une formation annulaire cunéiforme (74, 76) est prévue sur la surface circonférentielle de la première bague d'étanchéité (32), la formation annulaire (74, 76) étant située au voisinage de la surface d'étanchéité (44) du siège (32).

10. Appareil de fermentation (10) selon la revendication 9, **caractérisé en ce que** la formation annulaire (76) est formée d'un seul tenant avec la première bague d'étanchéité (32).

11. Appareil de fermentation (10) selon la revendication 10, **caractérisé en ce que** la formation annulaire (74) est formée séparément de la première bague d'étanchéité (32), en un matériau à faible coefficient de frottement.
